# EUROPEAN PATENT SPECIFICATION

(11) **EP 0 305 216 B1**
(45) Date of publication and mention of the grant of the patent: **02.08.1995**
(21) Application number: 88307980.8
(22) Date of filing: 26.08.1988
(51) Int. Cl.: C12N 9/20, C12N 9/28, C12N 15/80

(54) **Recombinant Humicola lipase and process for the production of recombinant humicola lipases**
Rekombinante Humicola-Lipase und Verfahren zur Herstellung von rekombinanten Humicola-Lipasen
Lipase recombinante de humicola et procédé de production de lipases recombinantes de humicola

(30) Priority: 28.08.1987 DK 4500/87; 15.12.1987 DK 6560/87; 15.04.1988 DK 2054/88
(43) Date of publication of application: 01.03.1989
(73) Proprietor: NOVO NORDISK A/S, 2880 Bagsvaerd (DK)
(72) Inventor: Boel, Esper, DK-2840 Holte (DK); Huge-Jensen, Ida Birgitte, DK-3630 Jaegerspris (DK)
(74) Representative: Thalsoe-Madsen, Kine Birgit

(56) References cited:
- EP-A- 0 184 438
- EP-A- 0 215 594
- EP-A- 0 238 023
- EP-A- 0 258 068
- BIOLOGICAL ABSTRACTS, vol. 56, no. 12, no. 68797, Philadelphia, PA, US; W. H. LIU et al.: "Purification and general properties of the lipase of thermophillic fungus Humicola lanuginosa S-38", & AGRIC. BIOL. CHEM. 37(1): 157-163,1973.
- CHEMICAL ABSTRACTS, vol. 83, 1975, page 401, abstract no. 112391y, Columbus, Ohio, US; & JP-A-75 63 188 (YAMASA SHOYU CO., LTD) 29-05-1975
- THE EMBO JOURNAL, vol. 4, no. 2, 1985, pages 475-479, IRL Press Ltd, Oxford, England; J.M KELLY et al.: "Transformation of Aspergillus niger by the amdS gene of Aspergillus nidulans"
- BIOCHEMICAL AND BIOPHYSICAL RESEARCH COMMUNICATIONS, vol. 141, no. 1, 26th November 1986, pages 185-190, Academic Press Inc.; W. KUGIMIYA et al.:"Molecular cloning and nucleotide sequence of the lipase gene from Pseudomonas fragi"

## Description

### BACKGROUND OF THE INVENTION

The present invention relates to a process for recombinant DNA production of Humicola lipases and a recombinant Humicola lipase.

Humicola lipases are obtainable from strains of thermophilic Humicola sp., including thermophilic Thermomyces sp., such as H. lanuginosa (Griffon and Maublanc) Bunce, H. stellata Bunce, H. grisea var. thermoidea, Cooney & Emerson, H. insolens, Cooney & Emerson, Thermomyces ibadanensis, Apinis & Eggins, H. hyalothermophila Moubasher, Mazen and Abdel-Hafez, H. grisea var. indica Subrahmanyam, H. brevis var. thermoidea, Subrahmanyam and Thirumalachar and H. brevispora Subrahmanyam and Thirumalachar.

H. lanuginosa has also been described under the synonyms Thermomyces lanuginosus Tsiklinsky, Sepedonium lanuginosum Griffon and Maublanc, Sepedonium thermaphilum cyclosporum and S. thermaphilum ovosporum Velich, Acremoniella sp. Rege, Acremoniella thermophila Curzi and Monotospora lanuginosa (Griffon and Maublanc) Mason.

Moreover, the species Scytalidium thermophilum (Cooney & Emerson) Austwich was by Hedger (1975, The ecology of thermophilic fungi in Indonesia. In Biodegradation et Humification. Rapport du 1^{er} Colloque Internationel - Nancy 1974 (ed. G. Kilbertius, O. Reisinger, A. Mourey & J.A. Cancela Da Fonseca), Sarreguemines: Pierron Editeur - 57206) considered to belong to Humicola insolens.

Production of a Humicola lanuginosa lipase is described in Japanese unexamined patent publication No. 48-62990, and in EP patent application No. 87307684.8. The latter also teaches use of this lipase in lipolytic detergent additives.

Due to the world wide use of enzyme additives in detergents and due to the fact that Humicola lipases have turned out to be superior to known detergent lipases both as regards detergency and stability, commercial interest in such lipases is high.

In the production of industrial enzymes yields have always been important for the profitability of the production process. The traditional way of improvement is to mutate the wild strain so as to obtain higher yielding mutants. By means of recombinant DNA technology a further possibility is to transform the gene for the desired product into a host microorganism capable of producing higher yields than the wild strain or with other favourable characteristics.

Accordingly, it is the aim of the present invention to develop a method for the production of Humicola lipases by recombinant DNA-technology.

In the past, numerous processes have been developed for the production of polypeptides or proteins by means of the recombinant DNA technology. The main interest has been concentrated on bacteria and yeast, e.g. E. coli, Bacillus subtilis and Saccharomyces cerevisiae being well characterized species as regards for instance expression and selection systems.

Besides the above mentioned microorganisms, filamentous fungi, notably Aspergillus sp. such as Aspergillus niger and Aspergillus oryzae, being well-characterized and widely used microorganisms for the commercial production of enzymes, are attractive candidates as host microorganisms for recombinant DNA vectors.

In the last few years different selection markers for the transformation of Aspergillus nidulans have been described and procedures have recently been developed for integrative transformation of the filamentous fungus Aspergillus nidulans for the purpose of investigation of the genetic and molecular processes controlling fungal cell differentiation.

Transformation of A. nidulans has been demonstrated by using plasmids containing the Neurospora crassa pyr-4 gene (Ballance, D.J. et al., Biochem.Biophys.Res.Commun., 112 (1983) 284-289), the A. nidulans amdS gene (Tilburn, J.G. et al., Gene 26 (1983) 205-221), the A. nidulans trpC gene (Yelton, M.M. et al., Proc.Natl.Acad.Sci. U.S.A., 81 (1984) 1470-1474) and the A. nidulans argB gene (John, M.A. and Peberdy J., Microb.Technol. 6 (1984) 386-389). The transforming DNA was found to be integrated into the host genome at rather low frequencies (typically < 1000 transformants/»g of DNA).

Transformation of Aspergillus niger with the amdS gene of A. nidulans was described (Kelly, J.M. and Hynes, M.J., EMBO Journal 4 (1985), 475-479) and amdS was shown to be a potential selection marker for use in transformation of Aspergillus niger that cannot grow strongly on acetamide as a sole nitrogen source. Transformation of Aspergillus niger using the argB gene of Aspergillus nidulans has also been described (Buxton, F. P. et al., Gene 37 (1985), 207-214).

A process for preparing transformants of Aspergillus niger is described in EP No. 0184,438A and EP No. 0215,594 and 0249,350 describe expression of heterologous polypetides in filamentous fungi. Finally WO87/04464 describes expression of higher eucaryotic genes in Aspergillus.

### BRIEF DESCRIPTION OF THE INVENTION

According to the present invention it has now been shown that it is possible to obtain a high level of expression of the Humicola sp. lipase in Aspergillus sp. strains or to enhance the production of the lipase in Humicola strains.

In its broadest aspect the present invention provides a method for the production of Humicola lipases comprising the steps of
(a) providing a suitable recombinant DNA cloning vector comprising DNA sequences encoding functions facilitating gene expression and a DNA sequence encoding the Humicola lipase;
(b) transforming a suitable host organism with the cloning vector from step (a);
(c) culturing the transformed host in a suitable culture medium and optionally recovering the lipase from the culture medium.

In a more narrow aspect of the present invention there is provided a process for the production of Humicola lipases in Aspergillus comprising the steps of:
(a) providing a recombinant DNA cloning vector system capable of integration into the genome of an Aspergillus host in one or more copies and comprising: DNA sequences encoding functions facilitating gene expression; a suitable marker for selection of transformants; and a DNA sequence encoding the Humicola lipase;
(b) transforming the Aspergillus host which does not harbour a functional gene for the chosen selection marker with the recombinant DNA cloning vector system from step a; and
(c) culturing the transformed Aspergillus host in a suitable culture medium and optionally recovering of the lipase from the culture medium.

According to a second aspect of the present invention there is provided a method for production of Humicola lipases in Aspergillus by which method an Aspergillus strain being transformed with a recombinant DNA cloning vector system as described above is cultured in a suitable culture medium and the lipase is recovered from the culture medium.

According to a third aspect of the invention there is provided a novel recombinant Humicola lipase product characterized by a difference in glycosylation from the native Humicola lipases previously known. That is, the nature and optionally the extent of glycosylation of the recombinant Humicola lipase of the present invention is different from the lipase obtained from the naturally occuring Humicola strains. The Humicola lipase product of this invention is furthermore characterized by an improved thermostability compared to the corresponding native Humicola lipase.

More specifically the novel recombinant Humicola lipase is characterized in that the carbohydrate content is of about the same level or is greater than the carbohydrate content in the native lipase whereas the nature of the glycosylation is different from the native Humicola lipase, i.e. the novel Humicola lipase contains other carbohydrates than the native lipase. The carbohydrate content may typically be from about 5 to about 30% (w/w), whereas the carbohydrate content in the native lipase is about 4.9% (w/w). More specifically the carbohydrate content may be in the range from about 5 to about 15% (w/w) and even more specifically it may be in the range from about 7.5% to about 8.5% (w/w).

The recombinant Humicola lipase product of this invention may be used as an enzymatic detergent additive for use in detergents in a similar way as the native lipase, i.e. as described in EP patent application No. 87307684.8.

As used in the present specification the term "recombinant Humicola lipase" is applied to Humicola lipase produced by culturing a microorganism transformed with the cDNA encoding the native Humicola lipase. The term "native Humicola lipase" is applied to the Humicola lipase obtained from natural sources of thermophilic Humicola sp., including thermophilic Thermomyces sp. described in the introductory part of the present specification.

It has been found that recombinant Humicola lipase from A. oryzae is not identical with native Humicola lipase, notwithstanding that the peptide sequence is the same. Apparently the host microorganism glycosylates the expressed polypetide to a different extent than the donor microorganism and with different sugar moieties. In A. niger the extent of glycosylation seems to be on the same level whereas the sugar moities seem to be of the same kind as in A. oryzae. Differentiation and identification of the native lipase and of the recombinant lipase is possible through measurement of their glycosylation. Thus, taking as exemplary the native Humicola lanuginosa lipase and the recombinant form of this lipase from an A. oryzae transformant, both lipases are N-glycosylated, but with different sugar residues. The native lipase does not have any galactose and has less mannose than the recombinant lipase and also is glycosylated to a different degree, the native lipase having carbohydrate moieties that add approximately 1500 Daltons to its molecular weight (about 5%) and the recombinant lipase having moieties that add about 2600 Daltons (about 8%). For details see Example 5 hereinafter.

The novel recombinant Humicola lipase according to the present invention may comprise from about 1 to about 12 mol galactose per mol lipase protein and more specifically from about 1 to about 6 mole galactose per mol lipase protein. The content of mannose may be from about 3 to about 20 and more specifically from about 3 to about 12 mol mannose per mol lipase protein. The recombinant Humicola lipase will typically comprise about 2 mol N-acetylglucosamine, from about 3 to about 20 mol mannose and from about 1 to about 12 mol galactose per mol lipase protein. More specifically the recombinant Humicola lipase will contain about 2 mol N-acetylglucoseamine, about 3 to about 12 mol mannose and about 1 to about 6 mol galactose per mol lipase product. Even more specifically the recombinant Humicola lipase will contain about 2 mol N-acetylglycoseamine, about 6 to about 9 mol mannose and about 2 to about 4 mol galactose per mol lipase protein.

The differences in glycosylation have some effect on the enzyme properties. In particular, the thermal stability of highly purified H. lanuginosa recombinant lipase is superior vis a vis the thermal stability of comparably purified native lipase. In addition, the stability of the pure recombinant lipase in the presence of an alkaline Bacillus protease (Esperase™) tested at 40°C and at 55°C is superior vis a vis comparably pure native lipase.

Characteristically, enzyme products, here lipase products, particularly extracellular lipase products contain other enzyme activities notably proteolytic activity and non-enzymatically active peptides and amino acids derived from culture broth constituents.

In the instance of the Humicola sp. lipase, the recombinant lipase product has been found to be thermally more stable than the comparable native lipase product. According to the data of the inventors hereof part of the improvement in thermal stability may be attributed to the different glycosylation, and another part may be attributed to the absence of the Humicola proteolytic activity in the recombinant lipase product. In the instance of the lipase native to H. lanuginosa a brief description thereof in U.S. patent 4,707,291 reports that a commercially available H. lanuginosa lipase product (Amano CE) contained a substantial level of proteolytic activity. The inventors' native H. lanuginosa lipase product contained a comparable level of proteolytic activity. At elevated temperatures in particular the protease can be expected to degrade other enzymes. The improvement in thermal stability attributable to absence of the protease is cumulative of the improvement attributable to different glycosylation (see Example 6).

As has already been pointed out the present invention provides a method for producing high yields of the Humicola lipases by cultivation of transformed Aspergillus strains, and in a preferred embodiment a method for production of the Humicola lanuginosa lipase by cultivation of a transformant A. oryzae or A. niger host cell. A. oryzae is the most preferred host microorganism.

A. oryzae has for years been used in commercial scale for the production of the TAKA-amylase enzyme and of proteolytic enzymes, and accordingly fermentation technology for cultivating this microorganism is well developed and the microorganism itself is approved for use in the food industry. The present invention offers the possibility of using A. oryzae for industrial production of large quantities of recombinant Humicola lipases.

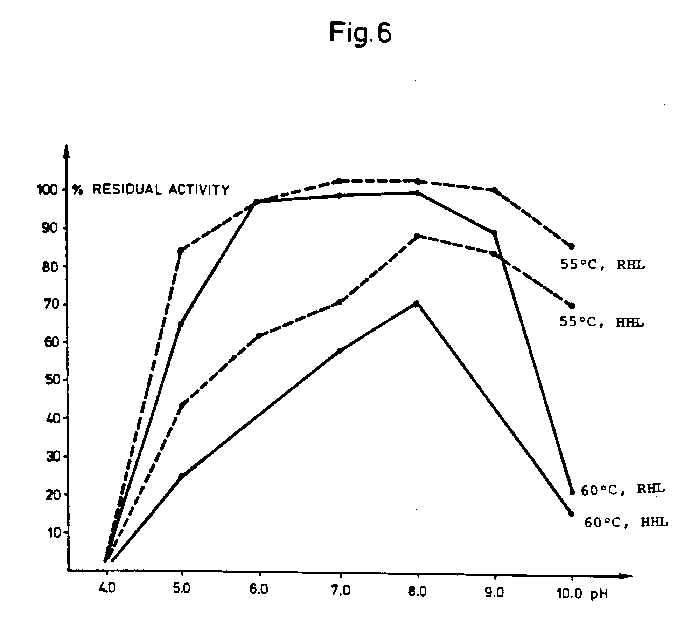

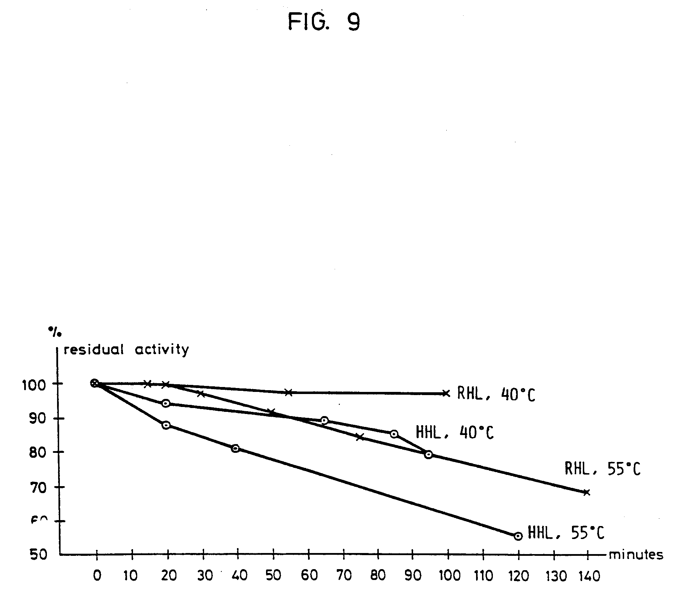

The present invention furthermore provides a recombinant Humicola lipase product superior to the native Humicola lipase for having increased thermostability, and increased stability in the presence of alkaline Bacillus proteases, see Figures 6 and 9 herein.

### BRIEF DESCRIPTION OF THE DRAWINGS

The present invention is further illustrated by reference to the accompanying drawings in which:
Fig. 1 shows the DNA-sequence of the TAKA-amylase promoter and upstream promoter regions, the preregion and the 5′part of the structural gene for the TAKA-amylase.
Fig. 2 shows an endonuclease restriction map of plasmid pTAKA17,
Fig. 3 illustrates the construction of plasmid pHLL,
Fig. 4 illustrates the construction of plasmid p960,
Fig. 5a and b shows the DNA sequence of prepro Humicola lanuginosa lipase cDNA together with the deduced amino acid sequence given by three-letter abbreviations,
Fig. 6 shows the residual activity of the recombinant Humicola lipase product compared to the residual activity of the native Humicola lipase product at 55°C and 60°C at different pH,
Fig. 7 shows an SDS-PAGE gradient gel,
Fig. 8 shows the residual activity at 55°C of the recombinant Humicola lipase product and the native Humicola lipase product along with the residual activity of the native Humicola lipase containing a protease inhibitor (PMSF), and
Fig. 9 shows the residual activity at 40°C and at 55°C of native and recombinant Humicola lipase in the presence of an alkaline Bacillus protease product (Esperase™)

### DETAILED DESCRIPTION OF THE INVENTION

The transformation technique used for the transformation of Aspergillus strains was a method adapted from the methods for transformation of A. nidulans (Ballance et al. Biochem.Biophys.Res.Commun., 112 (1983), 284-289; Tilburn et al., Gene 26 (1983), 205-221, Yelton et al. Proc.Natl.Acad.Sci. USA, 81 (1984), 1470-1474) and similar to the method of Buxton et al. (Gene 37 (1985), 207-214) for transformation of A. niger. In the process of the present invention an Aspergillus strain is transformed with a vector system containing a selection marker which is capable of being incorporated into the genome of the host strain, but which is not harboured in the host strain before the transformation. Transformants can then be selected and isolated from nontransformants on the basis of the incorporated selection marker.

Preferred selection markers are the argB (A. nidulans or A. niger), trpC (A. nidulans), amdS (A. nidulans), or pyr4 (Neurospora crassa) genes, or the DHFR (dihydrofolate reductase or mutants hereof) gene. More preferred selection markers are the argB or the amdS gene. Wild type A. oryzae strains are normally argB⁺ (i.e. the argB gene is functional in A. oryzae). If argB is chosen as the selection marker an argB mutant strain of A. oryzae which has a defect in the gene for this marker must be used as host strain. A. oryzae argB mutants can be prepared as described by F.P. Buxton et al. (Gene 37 (1985), 207-214). An argB mutant is defined as a mutant having a defect in the ornithin transcarbamylase gene. On the other hand the amdS gene may be used as selection marker for the transformation of wild type A. oryzae as the wild type strains do not contain this gene.

DNA-sequences encoding functions facilitating gene expression are typically promoters, transcription terminators and polyadenylation signals.

The promoter, which might be preceded by upstream activating sequences and enhancer sequences as well known in the art, may be any DNA-sequence that might show strong transcriptional activity in Aspergillus and may be derived from genes encoding both extracellular and intracellular proteins, such as amylases, glucoamylases, proteases, lipases, cellulases and glycolytic enzymes. Suitable promoters may be derived from genes for A. oryzae TAKA amylase, Rhizomucor miehei aspartic proteinase, A. niger glucoamylase, A. niger neutral α-amylase, A. niger acid stable α-amylase, and Rhizomucor miehei lipase. Examples of promoters from genes for glycolytic enzymes are TPI, ADH and PGK.

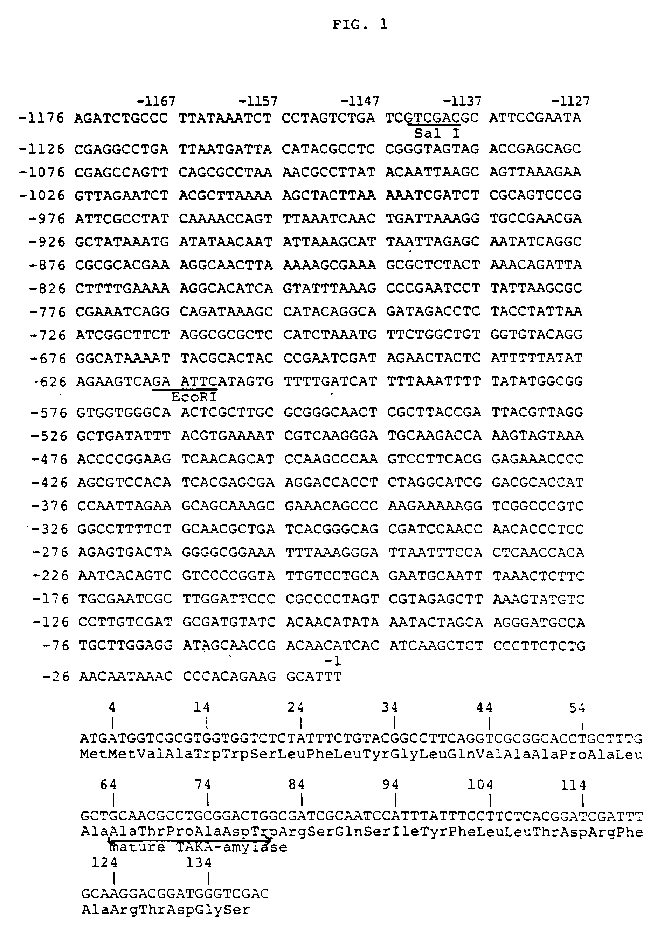

A preferred promoter according to the present invention is the A. oryzae TAKA-amylase promoter. The TAKA amylase is a well known α-amylase (Toda et al., Proc.Japan Acad. 58 Ser. B (1982) 208-212). DNA encoding the promoter region was derived from the TAKA-amylase genomic clone as described in EP patent application No. 87103806.3. The sequence of the promoter and regions upstream to the promoter together with the preregion and the 5′end of the structural gene for the TAKA-amylase is illustrated in Fig. 1.

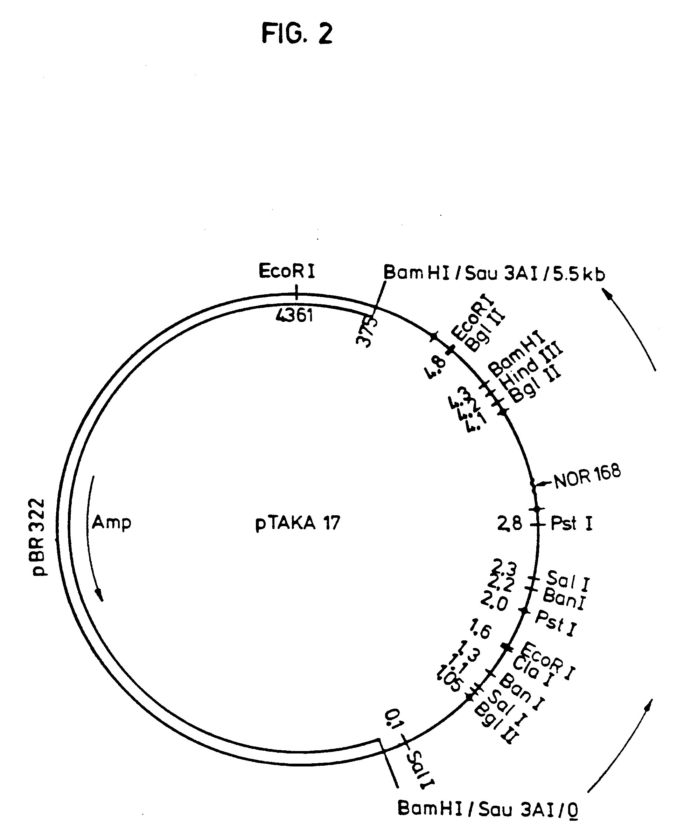

The TAKA-amylase promoter is available from plasmid pTAKA 17 being deposited in connection with EP patent application No. 87103806.3. The endonuclease restriction map of plasmid pTAKA17 is shown in fig. 2.

From pTAKA 17 the whole promoter sequence including sequences upstream to the promoter or functional parts thereof may be derived by means evident to the person skilled in the art. The promoter sequence may be provided with linkers with the purpose of introducing specific restriction sites facilitating the ligation of the promoter sequence with further DNA, for instance the gene encoding the desired protein product or different preregions (signal peptides).

In the method according to the present invention the sequence from nucleotide -1144 (see Fig. 1) (representing a SalI site) to nucleotide -10 has been used as one example of a well functioning part of the promoter region. In another embodiment of the present invention the nucleotide sequence from nucleotide -1176 to -1 was preceded by the still not sequenced 1.05 kb fragment from pTAKA 17. It is evident for the person skilled in the art that different fragments can be used.

According to one embodiment of the present invention the promoter and upstream activating sequences have the following sequence or a functionally equivalent nucleotide sequence: 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
CGCTTACCGA TTACGTTAGG GCTGATATTT ACGTGAAAAT CGTCAAGGGA
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
representing the sequence from nucleotide -1144 to -10 in Fig. 1.

According to a further embodiment the promoter and upstream activating sequences have the following sequence or a functionally equivalent nucleotide sequence: 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
representing the sequence from nucleotide -1176 to -1 in Fig. 1.

According to a further aspect of the present invention the latter sequence may be preceded by the 1.05 kb unsequenced upstream region from pTAKA 17 (position 0 to 1.05 in Fig. 2).

The terminators and polyadenylation sequences may be derived from the same sources as the promoters. Enhancer sequences may also be inserted into the construction.

The expressed product may be accumulated within the cells requiring disruption of the cells to isolate the product. To avoid this further process step and also to minimize the amount of possible degradation of the expressed product within the cells it is preferred that the product is secreted from the cells. For this purpose the gene for the desired product is provided with a preregion ensuring effective direction of the expressed product into the secretory pathway of the cell. This preregion which might be a naturally occuring signal or leader peptide or functional parts thereof or a synthetic sequence providing secretion is generally cleaved from the desired product during secretion leaving the mature product ready for isolation from the culture broth.

The preregion may be derived from genes for secreted proteins from any source of organism.

According to the present invention the preregion may be derived from a glucoamylase or an amylase gene from an Aspergillus species, an amylase gene from a Bacillus species, a lipase or proteinase gene from Rhizomucor miehei, the gene for the α-factor from S. cerevisiae or a Humicola lipase gene. More preferably the preregion may be derived from the gene for the Humicola lanuginosa lipase, A. oryzae TAKA amylase, A. niger neutral α-amylase, A. niger acid-stable α-amylase, B. licheniformis α-amylase, the maltogenic amylase from Bacillus NCIB 11837, B. stearothermophilus α-amylase or B. licheniformis subtilisin. The Humicola lanuginosa signal peptide has the following sequence

ATGAGGAGGTCCCTTGTGCTGTTCTTTGTCTCTGCGTGGACGGCCTTGGCC
MetArgSerSerLeuValLeuPhePheValSerAlaTrpThrAlaLeuAla

The TAKA-amylase signal has the following sequence 
ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla
The gene for the desired product functionally linked to promoter and terminator sequences may be incorporated in a vector containing the selection marker or may be placed on a separate vector or plasmid capable of being integrated into the genome of the host strain. As used herein the expression "vector system" includes a single vector or plasmid or two or more vectors or plasmids which together contain the total DNA-information to be integrated into the host genome. Vectors or plasmids may be linear or closed circular molecules. According to a preferred embodiment of the present invention the host organism is cotransformed with two vectors, one including the selection marker and the other comprising the remaining foreign DNA to be introduced in the host strain, including promoter, the gene or cDA for the desired product and transcription terminator and polyadenylation sequences.

Normally the transformants are stable and can be cultured in the absence of a selection pressure. If the transformants turn out to be unstable the selection marker may be used for selection during cultivation. The transformed cells are then cultured under a selection pressure corresponding to the marker in question.

Plasmids used as starting materials in the following examples are as follows:
- p285:: (ATCC No. 20681)
- pSal43:: Berse et al. Gene 25 (1983), 109-117 John & Peberdy, Enzyme Microb. Technol.
6 (1984), 386-389.
- p3SR2:: J.M. Kelly and M.J. Hynes, EMBO Journal 4 (1985), 475-479.
- pSP62-K2 and pCDVI-PL:: Noma et al. Nature, 319, (1986), 640-646)
- p775:: European patent application No. 87103806.3,
- pUC19:: Vieira et al., Gene 19 (1982), 259- 268 and Messing, Meth. in Enzymology 101 (1983), 20-27.

The strains used are as follows:
- A. oryzae*:: ATCC 20423, IFO 4177, ATCC 1011, ATCC 9576, ATCC 14488-11491, ATCC 11601 and ATCC 12892.
- E. coli:: MC1000 (Casabadan, M.J. and Cohen, S.N., J.Mol.Biol. 138, 179-207) (NCIB 11956)
- H. lanuginosa:: DSM 4109
- A. niger*:: ATCC 1015, ATCC 10582.

* ArgB mutants of these strains can be prepared as described by F.P. Buxton et al. (Gene 37 (1987) 207-214). An ArgB mutant is defined as a mutant having a defect in the ornithine transcarbamylase gene.

### Identification of Humicola lanuginosa lipase (HLL) amino acid sequence

In order to obtain information which allows the construction of a specific oligonucleotide probe, a partial sequence determination was carried out on the purified Humicola lanuginosa lipase. The supernatant from a culture broth of Humicola lanuginosa, from which mycelia and low molecular weight substances had been removed was subjected to a column chromatography performed by use of DEAE-sepharose (anion exchange chromatography), phenyl sepharose (hydrophobic interaction chromatography) followed by gel filtration on TSK G3000 SW. The sequence determination was performed with a Gas Phase Sequencer (Applied Biosystems Model 470A) as described by Thim, L. et al. (FEBS Lett. 212, 307-312 (1987).

The following N-terminal sequence was found: 
5                   10                  15
Glu-Val-Ser-Gln-Asp-Leu-Phe-Asn-Gln-Phe-Asn-Leu-Phe-Ala-Gln-
                20                  25
Tyr-Ser-Ala-Ala-Ala-Tyr-X-Gly-Lys-Asn-
This sequence allows the construction of two specific mixed oligonucleotide probes comprising the sequences from amino acid residue No. 7 - No. 11 (Phe-Asn-Gln-Phe-Asn) and amino acid residue No. 13 - No. 16 (Phe-Ala-Gln-Tyr), respectively. The screening of a Humicola lanuginosa cDNA library by use of HLL-specific oligonucleotide mixtures corresponding to the above sequences is described in example 1.

### Example 1

### Construction of a Humicola lanuginosa cDNA library in E. coli

Total RNA was extracted from homogenized H. lanuginosa mycelium using methods as described by Boel et al. (EMBO J., 3: 1097-1102, 1984) and Chirgwin et al., (Biochemistry (Wash.), 18, 5294-5299, 1979). Poly(A)-containing RNA was obtained by two cycles of affinity chromatography on oligo(dT)-cellulose as described by Aviv and Leder (PNAS, USA, 69, 1408-1412, 1972). cDNA was synthesized with the use of methods described by Okayama and Berg (Molec.Cell.Biol., 2: 161-170, 1982), and with the vectors pSP62-K2 and pCDVI-PL described by Noma et al. (Nature, 319: 640-646, 1986). The synthesized cDNA was transformed into a hsdR⁻, M⁺ derivative of E. coli MC1000 (Casadaban and Cohen, J. Mol.Biol., 138, 179-207, 1980) to generate recombinant clones.

### Identification of Humicola lanuginosa lipase (HLL) specific cDNA recombinants

A mixture of 32 pentadecamer oligodeoxyribonucleotides 
   A   A   A   A   A
d(  TT  AA  TG  TT  AA ),
   G   G   G   G   G
one of which is complementary to HLL mRNA in the region coding for Phe-Asn-Gln-Phe-Asn was synthesized on an Applied Biosystems, Inc. DNA synthesizer and purified by polyacrylamide gel electrophoresis. Approximately 10,000 E. coli recombinants from the Humicola lanuginosa cDNA library were transferred to Whatman 540 paper filters. The colonies were lysed and immobilized as described by Gergen et al. (Nucleic Acids Res. 7, 2115-2135, 1979). The filters were hybridized with the ³²P-labelled HLL-specific pentadecamer-mixture as described by Boel et al. (EMBO J. 3, 1097-1102, 1984). Hybridization and washing of the filters were done at 37°C and 43°C respectively, followed by autoradiography for 24 hours with an intensifier screen. Miniprep plasmid DNA was isolated from two hybridizing colonies, pHLL 702,3 and pHLL 702,4 by standard procedures (Birnboim and Doly, Nucleic Acids Res., 7, 1513-1523, 1979), and the DNA sequence of the cDNA insert was established by the procedure of Maxam and Gilbert (Methods Enzymol. 65, 499-560, 1980).

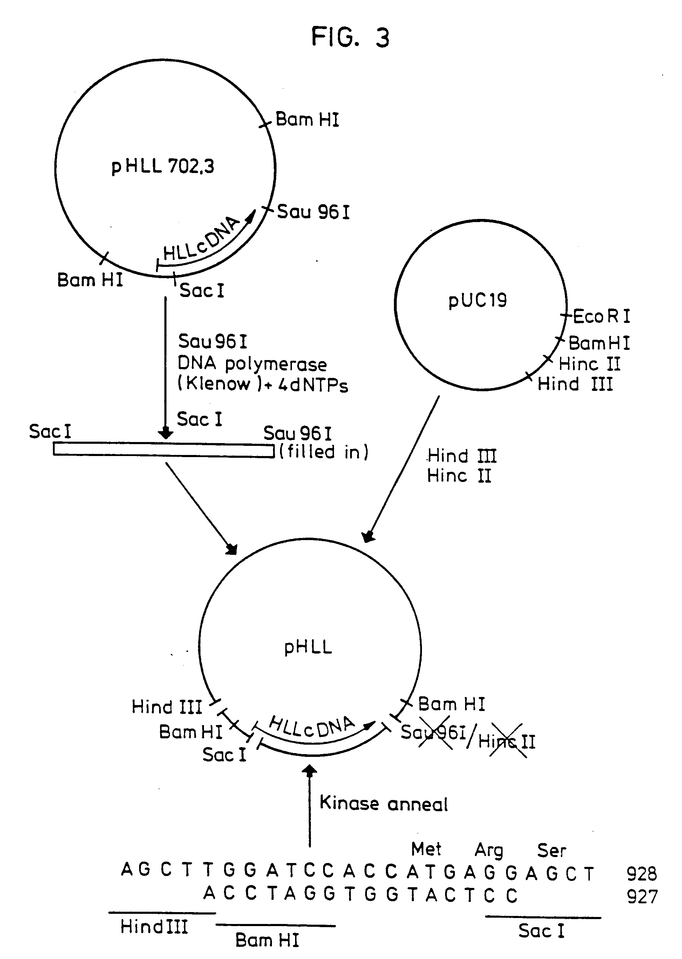

to facilitate further construction work with the HLL cDNA, DNA sequences containing unique restriction sites were added to the 5′ and the 3′ ends of the cDNA as follows. pHLL 702,3 was digested with Sau96I which digests HLL cDNA in the 3′untranslated region and the resulting ends were filled in with E. coli DNA polymerase (Klenow fragment) and the four dNTPs. This DNA was subsequentially digested with SacI which cuts the HLL cDNA once just 3′ to the initiating methionine codon. The resulting 0.9 kb HLL cDNA fragment was purified by agarose gel electrophoresis, electroeluted and made ready for ligation reactions. As a 5′ adaptor two oligonucleotides, 927 and 928, were synthesized. The sequence of the adaptor is shown in fig. 3. This adaptor was designed to add a HindIII and a BamHI site just 5′ to the initiating Met codon of HLL cDNA. The two oligos were kinased with ATP and T₄ polynucleotide kinase, annealed to each other and ligated to the purified 0.9 kb HLL cDNA sequence in a pUC19 vector that had been digested with HindIII and HincII and purified on a 0.7% agarose gel. The resulting plasmid pHLL carried the HLL cDNA as a portable 0.9 kb BamHI fragment. The construction of pHLL is shown in fig. 3.

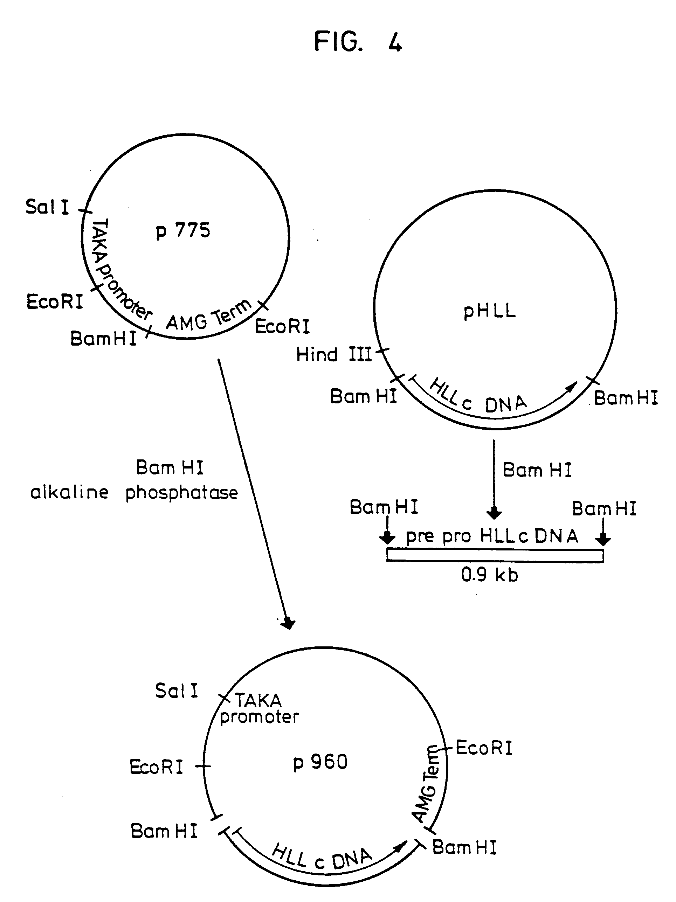

After BamHI digestion and purification of the 0.9 kb HLL cDNA fragment on an agarose gel it was ligated to BamHI digested and phosphatased p775 to generate p960 in which HLL cDNA is under transcriptional control of the TAKA promotor from Aspergillus oryzae and the AMG terminator from Aspergillus niger. The construction of p960 is shown in fig. 4.

The construction of p775 is described in European patent application No. 87103806.3.

p775 contains the TAKA promoter and AMG terminator and has a unique BamHI site as a cloning site.

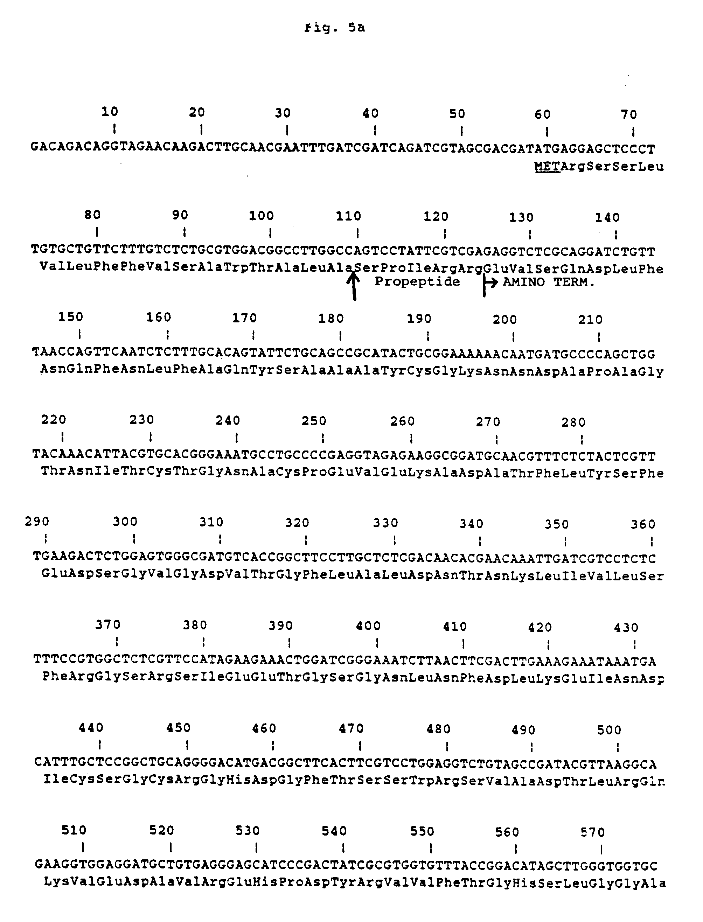

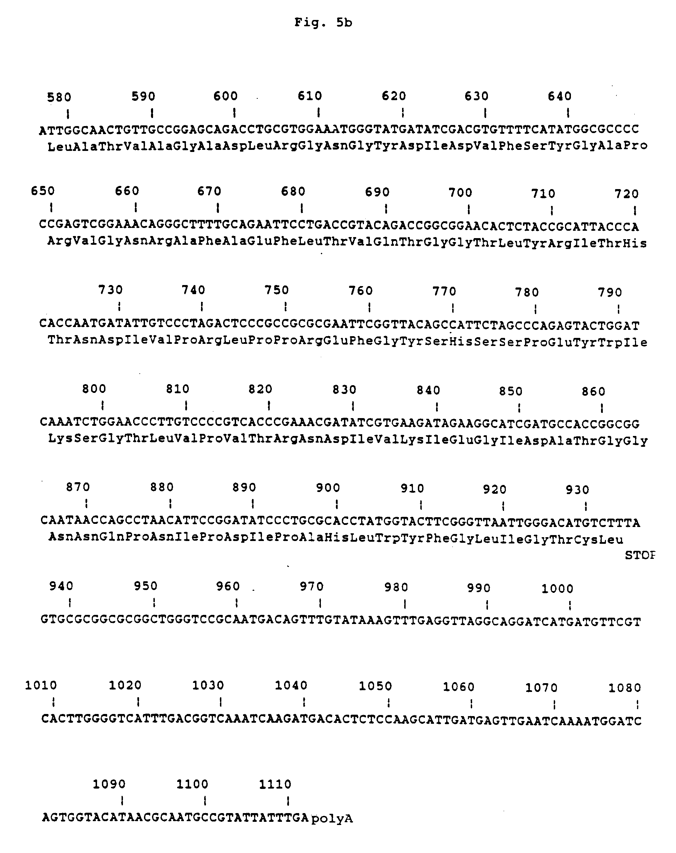

Figure 5a and b gives the sequence of prepro HLL cDNA with its deduced amino acid sequence. Nucleotides are numbered from the first base in the cloned cDNA. From this cDNA sequence it can be concluded that HLL is synthesized as a 291 amino acid residue long precursor with a signal peptide of 17 residues, and a short propeptide of 5 residues. The putative signal peptidase processing site (von Heijne, Eur.J.Biochem. 133, 17-21, 1983) is indicated with an arrow pointing to the peptide bond between an Ala and a Ser residue. The amino terminus of the mature enzyme as identified by amino terminal amino acid sequencing is indicated.

### Amino acid composition of Humicola lanuginosa (HLL)

Amino acid analysis was carried out by means of a Beckman Amino Acid Analyzer (Model JL1 MB) on samples (40 »g) previously hydrolyzed in sealed ampoules in 6 M HCl or 4 M methanesulfonic acid at 110°C for 24, 48 and 96 hours. Half-cystine was determined as S-β-(4-pyridylethyl)-cysteine after reduction by tributylphosphin followed by coupling with 4-vinylpyridine. All chemicals were of highest purity.

The results are shown in the following table and compared to the amino acid composition determined from cDNA sequencing.

| Amino Acid | Found | Nearest integer | cDNA |
|---|---|---|---|
| Ala | 20.85 | 21 | 21 |
| Arg | 14.42 | 14 | 14 |
| Asn | 36.91 | 37 | 19 |
| Asp | | | 19 |
| Cys^{c} | 5.54 | 6 | 6 |
| Gln | 18.20 | 18 | 6 |
| Glu | | | 12 |
| Gly | 27.51 | 28 | 28 |
| His | 5.94 | 6 | 6 |
| Ile^{b} | 14.99 | 15 | 16 |
| Leu | 20.06 | 20 | 20 |
| Lys | 7.05 | 7 | 7 |
| Met | 0 | 0 | 0 |
| Phe | 14.80 | 15 | 15 |
| Pro | 11.90 | 12 | 12 |
| Ser^{a} | 16.76 | 17 | 17 |
| Thr^{a} | 18.46 | 18 | 19 |
| Trp | 3.58 | 4 | 4 |
| Tyr | 9.84 | 10 | 10 |
| Val^{b} | 18.24 | 18 | 18 |

| | | | |
|---|---|---|---|
| a) extrapolated value to zero hydrolysis time | | | |
| b) extrapolated value to infinite hydrolysis time | | | |
| c) determined as S-β-(4-pyridylethyl)-cysteine. | | | |

### Example 2

### Transformation of Aspergillus oryzae or Aspergillus niger (general procedure)

100 ml of YPD (Sherman et al., Methods in Yeast Genetics, Cold Spring Harbor Laboratory, 1981) was inoculated with spores of A. oryzae, A. niger or argB mutants hereof and incubated with shaking at 37°C for about 2 days. The mycelium was harvested by filtration through miracloth and washed with 200 ml of 0.6 M MgSO₄. The mycelium was suspended in 15 ml of 1.2 M MgSO₄, 10 mM NaH₂PO₄, pH = 5.8. The suspension was cooled on ice and 1 ml of buffer containing 120 mg of Novozym® 234, batch 1687 was added. After 5 min., 1 ml of 12 mg/ml BSA (Sigma type H25) was added and incubation with gentle agitation continued for 1.5-2.5 hours at 37°C until a large number of protoplasts was visible in a sample inspected under the microscope.

The suspension was filtered through miracloth, the filtrate transferred to a sterile tube and overlayered with 5 ml of 0.6 M sorbitol, 100 mM Tris-HCl, pH = 7.0. Centrifugation was performed for 15 min. at 1000 g and the protoplasts were collected from the top of the MgSO₄ cushion. 2 volumes of STC (1.2 M sorbitol, 10 mM Tris-HCl pH = 7.5, 10 mM CaCl₂) were added to the protoplast suspension and the mixture was centrifugated for 5 min. at 1000 g. The protoplast pellet was resuspended in 3 ml of STC and repelleted. This was repeated. Finally the protoplasts were resuspended in 0.2-1 ml of STC.

100 »l of protoplast suspension was mixed with 5-25 »g of the appropriate DNA in 10 »l of STC. Protoplasts from the argB strains were mixed with pSal43 DNA (an A. nidulans argB gene carrying plasmid) and protoplasts from the argB⁺ strains were mixed with p3SR2 (an A. nidulans amdS gene carrying plasmid). The mixture was left at room temperature for 25 min. 0.2 ml of 60% PEG 4000 (BDH 29576), 10 mM CaCl₂ and 10 mM Tris-HCl pH = 7.5 was added and carefully mixed (twice) and finally 0.85 ml of the same solution was added and carefully mixed. The mixture was left at room temperature. for 25 min., spun at 2500 g for 15 min. and the pellet was resuspended in 2 ml of 1.2 M sorbitol. After one more sedimentation the protoplasts were spread on the appropriate plates. Protoplasts from the argB strains transformed with pSal43 were spread on minimal plates (Cove, Biochem.Biophys.Acta 113 (1966) 51-56) with glucose and urea as carbon and nitrogen sources respectively, and containing 1.2 M sorbitol for osmotic stabilization. Protoplasts from the argB⁺ strains transformed with p3SR2 were spread on minimal plates (Cove, Biochim.Biophys.Acta 113 (1966) 51-56) containing 1.0 M sucrose, pH = 7.0, 10 mM acetamide as nitrogen source and 20 mM CsCl to inhibit background growth. After incubation for 4-7 days at 37°C spores were picked, suspended in sterile water and spread for single colonies. this procedure was repeated and spores of a single colony after the second reisolation were stored as a defined transformant.

### Example 3

### Expression of recombinant Humicola lipase (RHL) in an A. oryzae strain

p960 is transformed into A. oryzae IFO 4177 by cotransformation with p3SR2 containing the amdS gene from A. nidulans as described with a mixture of equal amounts of p960 and p3SR2 (approximately 5 »g of each). Transformants which can use acetamide as sole nitrogen source are reisolated twice. After growth on YPD (Sherman et al, 1981) for three days culture supernatants are analysed by SDS-PAGE. The gels are stained with coomassie brilliant blue R. The best transformants are selected for further studies and grown in a 2 liter Kieler fermentor on 4% soy bean meal and supplied with glucose during growth. The culture is heavily agitated during fermentation. The recombinant Humicola lipase product (RHL) was isolated from the culture broth by removal of the cells by centrifugation, ultrafiltration of the supernatant and freeze drying.

### Example 4

### Expression of Humicola lipase in an A. niger strain

p960 was transformed into A. niger argB by cotransformation with pSal43 containing the argB gene form A. nidulans as described in Example 2. Protoplast were incubated with equal amounts, approximately 5»g, of each plasmid. Transformants were selected on minimal plates (Cove Biochim. Biophys.Acta 113 (1966), 55-56) by relief of argenine requirement.

After two reisolations of conidiospores the transformants were cultured for seven days in YPD (Sherman et al., 1981) at 30°C. The culture supernatants were analyzed by SDS-PAGE. Most of the transformants produced Humicola lipase in their supernatants.

The carbohydrate content was analyzed by Endo H treatment as described in Example 5. It was found that the carbohydrate content was of about the same magnitude as for the native Humicola lipase. The Endo H sensitivity was the same as for the recombinant Humicola lipase from A. oryzae. It was accordingly assumed that the nature of the glycosylation is the same in A. oryzae and A. niger.

### Example 5

Determination of carbohydrate content in RHL (recombinant lipase product from example 3) and HLL (native lipase product from cultivaton of DSM4109).

Treatment with enzymes capable of cleaving off carbohydrate side chains (Endo H and Glycopeptidase F).

RHL and HLL were treated with glycopeptidase F (Boehringer No. 91378, 100 units. 1 vial dissolved in 500 »l water) and Endo H (Sigma No. E6878, dissolved in citrate buffer pH 5.5).

HLL and RHL were each dissolved in 10 mM Tris pH 7.5 (1 mg/ml). To 100 »l HLL and RHL, respectively 2 »l glycopeptidase F was added and the samples were incubated at 37°C for 20 hours.

HLL and RHL were furthermore each dissolved in 10 mM Tris pH 7.5 (1 mg/ml) and 25 »l Endo H and 50 »l 0.1 M sodium acetate pH 5.0 were added to 25 »l of HLL and RHL, respectively. The samples were incubated at 37°C for 20 hours.

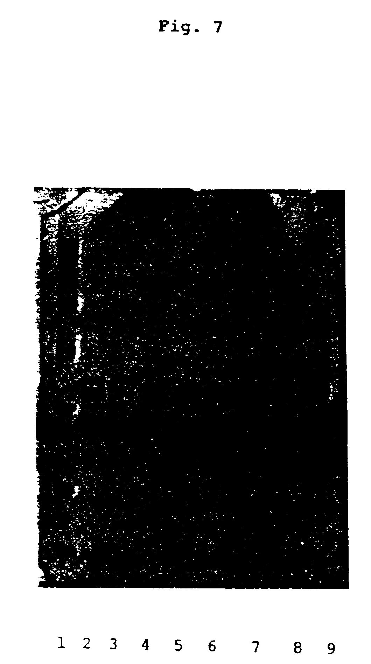

The samples were run on SDS-PAGE gradient gels 7.5-20% together with untreated HLL and RHL. The SDS-PAGE gradient gel is shown in fig. 7. The samples in fig. 7 are a follows:
1) Standard: 92K, 67K, 43K, 30K, 20.1K, 14.4K
2) HLL, 1 mg/ml in 10 mM Tris pH 7.5.
3) HLL + Endo H
4) RHL + Endo H
5) RHL + Glycopeptidase F
6) HLL + Glycopeptidase F
7) RHL + Endo H
8) RHL + Glycopeptidase F
9) RHL, 1 mg/ml in 10 mM Tris pH 7.5.

It appears from fig. 7 that glycopeptidase F is capable of cleaving off the carbohydrate part of both HLL and RHL and that the remaining protein is of the same size. Endo H is on the other hand only capable of cleaving off carbohydrates of RHL whereas Endo H treatment of HLL does not have any effect.

Accordingly, both HLL and RHL are N-glycosylated. The glycosylation is, however, of a different nature.

Carbohydrate analysis was performed by use of methods described by Thim et. al., submitted for publication in Biochemistry, Chaplin, M.F. (1982), Anal.Biochem. 123, 336-341, and Jentoft, N. (1985), Anal.Biochem. 148, 424-433.

Briefly, samples containing lipase was subjected to methanolysis followed by re-N-acetylation of the amino sugars and elimination of O-acetyl groups by a second, mild methanolysis step. Derivatives suitable for analysis by reverse phase high pressure liquid chromatography was obtained by perbenzoylation of the methyl glycosides.

The result of the analysis is shown in the following table.

| | Native Humicola lipase HLL mol/mol | Recombinant Humicola lipase RHL mol/mol |
|---|---|---|
| N-acetylglucosamine | 1.2 | 1.2 |
| Mannose | 5.7 | 8.6 |
| Galactose | 0 | 3.3 |

The carbohydrate moiety of the native Humicola lipase, HLL is composed of the two monosaccharides found in N-glycosylations of the high-mannose type (Montrenil, J. et al., (1986) in "Carbohydrate analysis: a practical approach", Chaplin, M.F. and Kenndey, J.F. (Eds.), IRL Press, Oxford, p 143). The result for N-acetylglucosamine (< 2 mol/mol) indicates that the primary sequence only contains a single N-glycosylation. This could further be deduced from the cDNA sequence (Fig. 5a + 5b). In addition mannose could be O-glycosidic linked to serine or threonine residues.

Besides N-acetylglucosamine and mannose, the recombinant Humicola lipase, RHL comprises galactose in significant amount. As for the native lipase the content of N-acetylglucosamine indicates the presence of a single N-glycosylation, although this is of the complex or hybrid type if galactose is part hereof. O-glycosylation at serine or threonine residues, however, has to be responsible for the presence of galactose if the N-glycosylation of the recombinant RHL lipase is of the high-mannose type as was the case for the native HLL lipase.

In average, the carbohydrate moieties add approximately 1500 D and 2600 D to the molecular weight of the native Humicola lipase HLL and the recombinant Humicola lipase RHL, respectively. This corresponds to a carbohydrate content of about 4.9% and 8.1%, respectively.

### Example 6

### Effect of pH and temperature on the stability of RHL and HLL

RHL (1.4 x 10⁶ LU/g) and HLL (0.2 x 10⁶ LU/g), respectively, were dissolved in buffer solutions of various pH and incubated for two hours at temperatures 55° and 60°C.

The buffer solutions were made up of 47,5 mM Na-acetate, MOPS (3-(N-morpholino)-propane sulphonic acid), and boric acid, with pH-adjustments to 4, 5, 6, 7, 8, 9, and 10 by means of 1 N HCl or 1 N NaOH. In addition the buffer solution used in a separate test of a sample of HLL that had been purified to 1.5 x 10⁶ LU/g was added PMSF (phenylmethan sulfonyl fluorid) to inhibit protease content in this native lipase product.

Lipase concentrations in the solutions were equated to approx. 10-15 LU/ml.

Immediately after the incubations the lipase solutions were cooled down in an ice-water bath and kept there until analysis the same day (LU-method, AF 95).

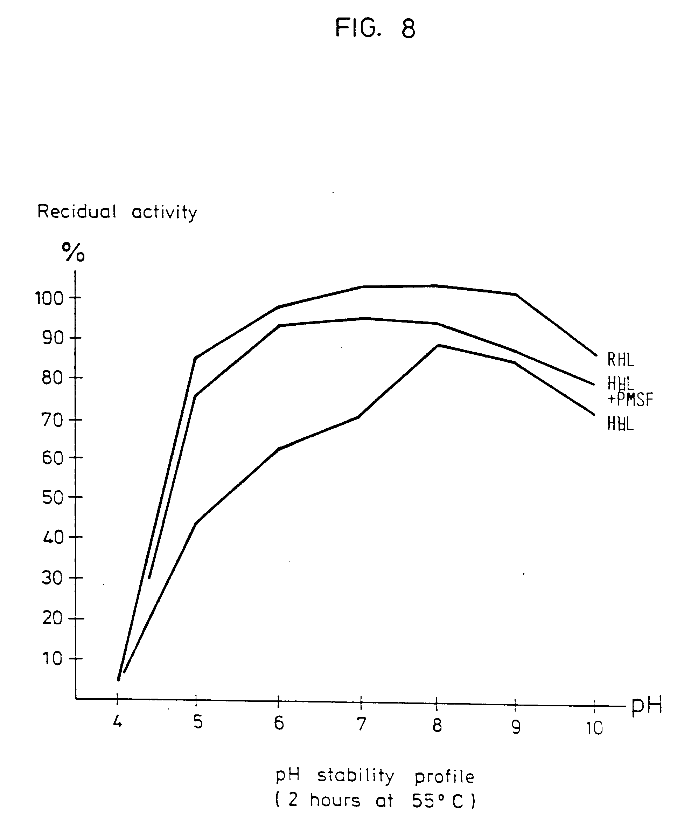

The results are given in fig. 6 and in fig. 8.

It appears from fig. 6 that the thermostability of RHL is greater than that of HLL at pH 5-10 both at 55°C and 60°C, respectively.

It appears from fig. 8 that the greater thermostability of RHL is due only in part to absence of the H. lanuginose protease activity.

### Example 7

Stability of RHL and HLL of comparable unit activity levels of 4 x 10⁶ LU/g in the presence of a typical alkaline Bacillus sp. protease, (here the commercially available detergent enzyme Esperase™).

The incubation conditions were 0.1 M boric acid, pH 9.5 and 40°C or 55°C using concentrations of 3600 LU/liter and 0.057 AU/liter (AU/LU = 0.016 x 10⁻³). The residual activities were calculated on the basis of reference incubations.

The results are given in Figure 9.

It appears from Fig. 9 that the thermostability of RHL in presence of an alkaline Bacillus protease is superior to that of HLL, indicating thereby differences in their susceptibility to attack by proteases.

The features disclosed in the foregoing description, in the following claims and/or in the accompanying drawings may, both separately and in any combination thereof, be material for realising the invention in diverse forms thereof.

## Claims

1. A process for producing a recombinant Humicola lipase in an Aspergillus strain, the process comprising the steps of
a) cultivating an Aspergillus sp. host transformed with a recombinant DNA cloning vector system, said vector system comprising DNA sequences including a promoter, transcription initiation sites, and transcription terminator and polyadenylation functions, and a DNA sequence encoding the Humicola sp. lipase, and
b) recovering said recombinant lipase from the culture.

2. A process according to claim 1, wherein the promoter is preceded by upstream activating sequences.

3. A process according to claim 1, wherein the DNA vector system further comprises a DNA sequence encoding a suitable marker for selection of transformants.

4. A process according to claim 3, wherein the selection marker is derived from the gene for A. nidulans or A. niger argB, A. nidulans trpC, A. nidulans amdS, Neurospora crassae Pyr4 or DHFR.

5. A process according to claim 4, wherein the selection marker is the argB gene derived from A. nidulans or A. niger or the amdS gene derived from A. nidulans.

6. A process according to claim 2, wherein the promoter and upstream activating sequences are derived from a gene encoding an extracellular or intracellular protein, such as an amylase, a glucoamylase, a protease, a lipase, a cellulase or a glycolytic enzyme.

7. A process according to claim 6, wherein the promoter and upstream activating sequences are derived from the gene for A. oryzae TAKA amylase, Rhizomucor miehei aspartic proteinase, A. niger neutral α-amylase, A. niger acid stable α-amylase, A. niger glucoamylase or Rhizomucor miehei lipase.

8. A process according to claim 1, wherein the Aspergillus host is an Aspergillus oryzae strain.

9. A process according to claim 8, wherein the promoter is the A. oryzae TAKA amylase promoter.

10. A process according to claim 9, wherein the promoter and upstream activating sequences have the following sequence: 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
 
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
 or a functional part thereof.

11. A process according to claim 9, wherein the promoter and upstream activating sequences have the following sequence: 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
CGCTTACCGA TTACGTTAGG GCTGATATTT ACGTGAAAAT CGTCAAGGGA
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
 
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
 or a functional part thereof.

12. A process according to claim 9, wherein the sequence in claim 10 is preceded by the 1.05 kb unsequenced upstream region from position 0 to 1.05 in plasmid pTAKA 17.

13. A process according to claim 1, wherein the vector system further comprises a signal sequence operatively linked to the DNA sequence encoding said Humicola lipase.

14. A process according to claim 13, wherein the signal sequence is derived from a glucoamylase or an amylase gene from an Aspergillus species, an amylase gene from a Bacillus species, a lipase or proteinase gene from Rhizomucor miehei, the gene for the α-factor from S. cerevisiae or a Humicola lipase gene.

15. A process according to claim 14, wherein the signal sequence is derived from the gene for the Humicola lanuginosa lipase, the gene for A. oryzae TAKA amylase, A. niger neutral α-amylase, A. niger acid-stable α-amylase, B. licheniformis α-amylase, the maltogenic amylase from Bacillus licheniformis subtilisin.

16. A process according to claim 3, wherein the vector system comprises two vectors, where one contains the selection marker and the other contains DNA sequences comprising a promoter, transcription initiation sites, and transcription termination and polyadenylation functions, and a DNA sequence encoding the Humicola lipase.

17. The process of claim 1, wherein the DNA sequence encoding a Humicola lipase encodes a Humicola lanuginosa lipase.

18. A recombinant Humicola sp. lipase, wherein the carbohydrate content is from 5 to 30% (w/w).

19. A recombinant Humicola sp. lipase according to claim 18, wherein the carbohydrate content is from 5 to 15% (w/w).

20. Recombinant Humicola sp. lipase according to claim 19, wherein the carbohydrate content is from about 6 to about 10% (w/w).

21. Recombinant Humicola sp. lipase according to claim 20, wherein the carbohydrate content is from about 7.5 to about 8.5% (w/w).

22. A recombinant Humicola sp. lipase, characterized in that it comprises galactose.

23. Recombinant Humicola sp. lipase according to claim 22, characterized in that it comprises N-acetylglucosamine, mannose and galactose.

24. Recombinant Humicola sp. lipase according to claim 22, characterized in that it comprises from about 1 to about 12 mol galactose per mol lipase protein.

25. Recombinant Humicola sp. lipase according to claim 24, characterized in that it comprises from about 1 to about 6 mol galactose per mol lipase protein.

26. Recombinant Humicola sp. lipase, characterized in that it comprises from about 3 to about 20 mol mannose per mol lipase protein.

27. Recombinant Humicola sp. lipase according to claim 26, characterized in that it comprises from about 3 to about 12 mol mannose per mol lipase protein.

28. Recombinant Humicola sp. lipase according to any of claims 18-27, characterized in that it per mol lipase protein comprises about 2 mol N-acetylglucosamine, from about 3 to about 20 mol mannose and from about 1 to about 12 mol galactose.

29. Recombinant Humicola sp. lipase according to claim 28, characterized in that it per mol lipase protein comprises about 2 mol N-acetylglucosamine, about 3 to about 12 mol mannose and about 1 to about 6 mol galactose.

30. Recombinant Humicola sp. lipase according to claim 29, characterized in that it per mol lipase protein comprises about 2 mol N-acetylglucosamine from about 6 to about 9 mol mannose and from about 2 to about 4 mol galactose.

31. Recombinant Humicola sp. lipase, characterized in having a residual activity after 2 house at 60°C of at least 90% at pH from about 6 to about 9; or a residual activity after 2 hours at 60°C of at least 80% at pH from about 5.5 to about 9.2; or a residual activity after 2 hours at 55°C of at least 95% at pH from about 6 to about 9.5; or a residual activity at 55°C at pH 9 of at about 99%; or a residual activity at 55°C at pH 10 of at least 85%.

32. A recombinant Humicola sp. lipase according to any of claims 18-31, which is a Humicola lanuginosa lipase.

## Patentansprüche

1. Ein Verfahren zur Herstellung einer rekombinanten Humicola-Lipase in einem Aspergillus-Stamm, wobei das Verfahren die Schritte umfaßt
a) Kultivieren eines Aspergillus sp.-Wirtes, der mit einem rekombinanten DNA-Klonierungsvektorsystem transformiert worden ist, wobei besagtes Vektorsystem DNA-Sequenzen, die einen Promotor, Transkriptionsinitiationsstellen und Transkriptionsterminator- und Polyadenylierungsfunktionen einschließen, und eine DNA-Sequenz, die die Humicola sp.-Lipase kodiert, umfaßt, und
b) Gewinnen besagter rekombinanten Lipase aus der Kultur.

2. Ein Verfahren nach Anspruch 1, wobei dem Promotor flußaufwärtige aktivierende Sequenzen vorangehen.

3. Ein Verfahren nach Anspruch 1, wobei das DNA-Vektorsystem ferner eine DNA-Sequenz umfaßt, die einen geeigneten Marker für die Selektion von Transformanten kodiert.

4. Ein Verfahren nach Anspruch 3, wobei der Selektionsmarker abgeleitet ist vom Gen für A. nidulans oder A. niger argB, A. nidulans trpC, A. nidulans amdS, Neurospora crassae Pyr4 oder DHFR.

5. Ein Verfahren nach Anspruch 4, wobei der Selektionsmarker das argB-Gen, das von A. nidulans oder A. niger abgeleitet ist, oder das amdS-Gen, das von A. nidulans abgeleitet ist, ist.

6. Ein Verfahren nach Anspruch 2, wobei der Promotor und die flußaufwärtigen aktivierenden Sequenzen abgeleitet sind von einem Gen, das ein extrazelluläres oder intrazelluläres Protein kodiert, wie etwa eine Amylase, eine Glucoamylase, eine Protease, eine Lipase, eine Cellulase oder ein glykolytisches Enzym.

7. Ein Verfahren nach Anspruch 6, wobei der Promotor und die flußaufwärtigen aktivierenden Sequenzen abgeleitet sind vom Gen für A. oryzae, TAKA-Amylase, Rhizomucor miehei, Asparaginsäure-Proteinase, neutrale A. niger-α-Amylase, säurestabile A. niger-α-Amylase, A. niger-Glukoamylase oder Rhizomucor miehei-Lipase .

8. Ein Verfahren nach Anspruch 1, wobei der Aspergillus-Wirt ein Aspergillus oryzae-Stamm ist.

9. Ein Verfahren nach Anspruch 8, wobei der Promotor der A. oryzae-TAKA-Amylase-Promotor ist.

10. Ein Verfahren nach Anspruch 9, wobei der Promotor und die flußaufwärtigen aktivierenden Sequenzen die folgende Sequenz besitzen: 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
 oder einen funktionellen Teil derselben.

11. Ein Verfahren nach Anspruch 9, wobei der Promotor und die flußaufwärtigen aktivierenden Sequenzen folgende Sequenz besitzen: 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
CGCTTACCGA TTACGTTAGG GCTGATATTT ACGTGAAAAT CGTCAAGGGA
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
 oder einen funktionellen Teil derselben.

12. Ein Verfahren nach Anspruch 9, wobei der Sequenz in Anspruch 10 die 1,05 kb nicht-sequenzierte flußaufwärtige Region von Position 0 bis 1.05 in Plasmid pTAKA17 vorangeht.

13. Ein Verfahren nach Anspruch 1, wobei das Vektorsystem ferner eine Signalsequenz umfaßt, die operativ verknüpft ist mit der DNA-Sequenz, die besagte Humicola-Lipase kodiert.

14. Ein Verfahren nach Anspruch 13, wobei die Signalsequenz abgeleitet ist von einem Glucoamylase- oder einem Amylase-Gen aus einer Aspergillus-Spezies, einem Amylase-Gen aus einer Bacillus-Spezies, einem Lipase- oder Proteinase-Gen aus Rhizomucor miehei, dem Gen für den α-Faktor aus S. cerevisiae oder einem Humicola-Lipase-Gen.

15. Ein Verfahren nach Anspruch 14, wobei die Signalsequenz abgeleitet ist vom Gen für die Lipase in Humicola lanuginosa, dem Gen für A. oryzae-TAKA-Amylase, neutrale A. niger-α-Amylase A.niger, säurestabile A. niger-α-Amylase, B. licheniformis-α-Amylase, die maltogene Amylase aus Bacillus licheniformis subtilisin.

16. Verfahren nach Anspruch 3, wobei das Vektorsystem zwei Vektoren umfaßt, von denen einer den Selektionsmarker enthält und der andere DNA-Sequenzen, die einen Promotor, Transkriptionsinitiationsstellen und Transkriptionsterminations- und Polyadenylierungsfunktionen umfassen, und eine DNA-Sequenz, die die Humicola-Lipase kodiert, enthält.

17. Das Verfahren nach Anspruch 1, wobei die DNA-Sequenz, die eine Humicola-Lipase kodiert, eine Humicola lanuginosa-Lipase kodiert.

18. Eine rekombinante Humicola sp.-Lipase, wobei der Kohlehydratgehalt von 5 bis 30 % (w/w) beträgt.

19. Eine rekombinante Humicola sp.-Lipase gemäß Anspruch 18, wobei der Kohlehydratgehalt von 5 bis 15 % (w/w) beträgt.

20. Rekombinante Humicola sp.-Lipase nach Anspruch 19, wobei der Kohlehydratgehalt von etwa 6 bis etwa 10 % (w/w) beträgt.

21. Rekombinante Humicola sp.-Lipase nach Anspruch 20, wobei der Kohlehydratgehalt von etwa 7,5 bis etwa 8,5 % (w/w) beträgt.

22. Eine rekombinante Humicola sp.-Lipase, die dadurch gekennzeichnet ist, daß sie Galactose umfaßt.

23. Rekombinante Humicola sp.-Lipase nach Anspruch 22, dadurch gekennzeichnet, daß sie N-Acetylglucosamin, Mannose und Galactose umfaßt.

24. Rekombinante Humicola sp.-Lipase nach Anspruch 22, dadurch gekennzeichnet, daß sie von etwa 1 bis etwa 12 mol Galactose pro mol Lipase-Protein umfaßt.

25. Rekombinante Humicola sp.-Lipase nach Anspruch 24, dadurch gekennzeichnet, daß sie von etwa 1 bis etwa 6 mol Galactose pro mol Lipase-Protein umfaßt.

26. Rekombinante Humicola sp.-Lipase, dadurch gekennzeichnet, daß sie von etwa 3 bis etwa 20 mol Mannose pro mol Lipase-Protein umfaßt.

27. Rekombinante Humicola sp.-Lipase nach Anspruch 26, dadurch gekennzeichnet, daß sie von etwa 3 bis etwa 12 mol Mannose pro mol Lipase-Protein umfaßt.

28. Rekombinante Humicola sp.-Lipase nach den Ansprüchen 18-27, dadurch gekennzeichnet, daß sie pro mol Lipase-Protein etwa 2 mol N-Acetylglucosamin, von etwa 3 bis etwa 20 mol Mannose und von etwa 1 bis etwa 12 mol Galactose umfaßt.

29. Rekombinante Humicola sp.-Lipase nach Anspruch 28, dadurch gekennzeichnet, daß sie pro mol Lipase-Protein etwa 2 mol N-Acetylglucosamin, etwa 3 bis etwa 12 mol Mannose und etwa 1 bis 6 mol Galactose umfaßt.

30. Rekombinante Humicola sp.-Lipase nach Anspruch 29, dadurch gekennzeichnet, daß sie pro mol Lipase-Protein etwa 2 mol N-Acetylglucosamin, von etwa 6 bis etwa 9 mol Mannose und von etwa 2 bis etwa 4 mol Galactose umfaßt.

31. Rekombinante Humicola sp.-Lipase, dadurch gekennzeichnet, daß sie eine Restaktivität nach 2 Stunden bei 60°C von wenigstens 90 % bei einem pH von etwa 6 bis etwa 9; oder eine Restaktivität nach 2 Stunden bei 60°C von wenigstens 80 % bei einem pH von etwa 5,5 bis etwa 9,2; oder eine Restaktivität nach 2 Stunden bei 55°C von wenigstens 95 % bei einem pH von etwa 6 bis etwa 9,5; oder eine Restaktivität bei 55°C bei pH 9 von wenigstens 99 % oder eine Restaktivität bei 55°C bei pH 10 von wenigstens 85 % besitzt.

32. Eine rekombinante Humicola sp.-Lipase nach einem der Ansprüche 18-31, die eine Humicola lanuginosa-Lipase ist.

## Revendications

1. Procédé de production d'une lipase d'*Humicola* recombinée dans une souche d'*Aspergillus*, ce procédé comprenant les étapes selon lesquelles
a) on cultive un hôte *Aspergillus sp*. transformé avec un système de vecteurs de clonage d'ADN recombiné, ledit système de vecteurs comprenant des séquences d'ADN comprenant un promoteur, des sites d'initiation de la transcription, et des fonctions de terminaison de la transcription et de polyadénylation, et une séquence d'ADN codant pour la lipase d'*Humicola sp*., et
b) on récupère ladite lipase recombinée à partir de la culture.

2. Procédé selon la revendication 1, dans lequel le promoteur est précédé par des séquences activatrices en amont.

3. Procédé selon la revendication 1, dans lequel le système de vecteurs d'ADN comprend en outre une séquence d'ADN codant pour un marqueur convenable pour la sélection des transformants.

4. Procédé selon la revendication 3, dans lequel le marqueur de sélection est dérivé du gène argB d'*A*. *nidulans* ou d'*A*. *niger*, trpC d'*A*. *nidulans*, amdS d'*A*. *nidulans* , Pyr4 de *Neurospora crassae* ou du DHFR.

5. Procédé selon la revendication 4, dans lequel le marqueur de sélection est le gène *argB* dérivé d'*A*. *Nidulans* ou d'*A*. *niger* ou le gène *amdS* dérivé d'*A*. *nidulans*.

6. Procédé selon la revendication 2, dans lequel le promoteur et les séquences activatrices en amont dérivent d'un gène codant pour une protéine extracellulaire ou intracellulaire, comme une amylase, une glucoamylase, une protéase, une lipase, une cellulase ou une enzyme glycolytique.

7. Procédé selon la revendication 6, dans lequel le promoteur et les séquences activatrices en amont dérivent du gène codant pour l'amylase TAKA d'*A*. *oryzae*, la protéinase aspartique de *Rhizomucor miehei*, l'α-amylase neutre d'*A*. *niger*, l'α-amylase stable aux acides d'*A*. *niger*, la glucoamylase d'*A*. *niger* ou la lipase de *Rhizomucor miehei*.

8. Procédé selon la revendication 1, dans lequel l'hôte *Aspergillus* est une souche d'*Aspergillus oryzae*.

9. Procédé selon la revendication 8, dans lequel le promoteur est le promoteur de l'amylase TAKA d'*A*. *oryzae*.

10. Procédé selon la revendication 9, dans lequel le promoteur et les séquences activatrices en amont ont la séquence suivante: 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
 ou une partie fonctionnelle de cette séquence.

11. Procédé selon la revendication 9, dans lequel le promoteur et les séquences activatrices en amont ont la séquence suivante: 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
 
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
CGCTTACCGA TTACGTTAGG GCTGATATTT ACGTGAAAAT CGTCAAGGGA
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
 ou une partie fonctionnelle de cette séquence.

12. Procédé selon la revendication 9, dans lequel la séquence de la revendication 10 est précédée de la région en amont non séquencée de 1,05 kb de la position 0 à la position 1,05 du plasmide pTAKA 17.

13. Procédé selon la revendication 1, dans lequel le système de vecteurs comprend en outre une séquence signal liée de façon opérationnelle à la séquence d'ADN codant pour ladite lipase d'*Humicola*.

14. Procédé selon la revendication 13, dans lequel la séquence signal dérive d'un gène de glucoamylase ou d'amylase d'une espèce d'*Aspergillus*, d'un gène d'amylase d'une espèce de *Bacillus*, d'un gène de lipase ou de protéinase de *Rhizomucor miehei*, du gène codant pour le facteur α de *S*. *cerevisiae* ou d'un gène de lipase d'*Humicola*.

15. Procédé selon la revendication 14, dans lequel la séquence signal dérive du gène codant pour la lipase d'*Humicola lanuginosa*, du gène codant pour l'amylase TAKA d'*A*. *oryzae* , l'α-amylase neutre d'*A*. *niger* , l'α-amylase stable aux acides d'*A*. *niger* , l'α-amylase de *B*. *licheniformis*, l'amylase maltogène de *B*. *licheniformis subtilisin*.

16. Procédé selon la revendication 3, dans lequel le système de vecteurs comprend deux vecteurs, dont l'un contient le marqueur de sélection et l'autre contient des séquences d'ADN comprenant un promoteur, des sites d'initiation de la transcription, et des fonctions de terminaison de la transcription et de polyadénylation, et une séquence d'ADN codant pour la lipase d'*Humicola*.

17. Procédé selon la revendication 1, dans lequel la séquence d'ADN codant pour une lipase d'*Humicola* code pour une lipase d'*Humicola lanuginosa*.

18. Lipase d'*Humicola sp*. recombinée, dans laquelle la teneur en hydrates de carbone est comprise entre 5 et 30 % (en masse).

19. Lipase d'*Humicola sp*. recombinée selon la revendication 18, dans laquelle la teneur en hydrates de carbone est comprise entre 5 et 15 % (en masse).

20. Lipase d'*Humicola sp*. recombinée selon la revendication 19, dans laquelle la teneur en hydrates de carbone est comprise entre environ 6 et environ 10 % (en masse).

21. Lipase d'*Humicola sp*. recombinée selon la revendication 20, dans laquelle la teneur en hydrates de carbone est comprise entre environ 7,5 et environ 8,5 % (en masse).

22. Lipase d'*Humicola sp*. recombinée, caractérisée en ce qu'elle contient du galactose.

23. Lipase d'*Humicola sp*. recombinée selon la revendication 22, caractérisée en ce qu'elle contient de la N-acétylglucosamine, du mannose et du galactose.

24. Lipase d'*Humicola sp*. recombinée selon la revendication 22, caractérisée en ce qu'elle contient d'environ 1 à environ 12 moles de galactose par mole de protéine lipase.

25. Lipase d'*Humicola sp*. recombinée selon la revendication 24 caractérisée en ce qu'elle contient d'environ 1 à environ 6 moles de galactose par mole de protéine lipase.

26. Lipase d'*Humicola sp*. recombinée, caractérisée en ce qu'elle contient d'environ 3 à environ 20 moles de mannose par mole de protéine lipase.

27. Lipase d'*Humicola sp*. recombinée selon la revendication 26, caractérisée en ce qu'elle contient d'environ 3 à environ 12 moles de mannose par mole de protéine lipase.

28. Lipase d'*Humicola sp*. recombinée selon l'une quelconque des revendications 18 à 27, caractérisée en ce qu'elle contient, par mole de protéine lipase, environ 2 moles de N-acétylglucosamine, d'environ 3 à environ 20 moles de mannose et d'environ 1 à environ 12 moles de galactose.

29. Lipase d'*Humicola sp*. recombinée selon la revendication 28, caractérisée en ce qu'elle contient, par mole de protéine lipase, environ 2 moles de N-acétylglucosamine, d'environ 3 à environ 12 moles de mannose et d'environ 1 à environ 6 moles de galactose.

30. Lipase d'*Humicola sp*. recombinée selon la revendication 29, caractérisée en ce qu'elle contient, par mole de protéine lipase, environ 2 moles de N-acétylglucosamine, d'environ 6 à environ 9 moles de mannose et d'environ 2 à environ 4 moles de galactose.

31. Lipase d'*Humicola sp*. recombinée, caractérisée en ce qu'elle a une activité résiduelle d'au moins 90 % après 2 heures à 60°C à un pH d'environ 6 à environ 9; ou une activité résiduelle d'au moins 80 % après 2 heures à 60°C à un pH d'environ 5,5 à environ 9,2; ou une activité résiduelle d'au moins 95 % après 2 heures à 55°C à un pH d'environ 6 à environ 9,5; ou une activité résiduelle d'au moins 99 % à 55°C à pH 9; ou une activité résiduelle d'au moins 85 % à 55°C à pH 10.

32. Lipase d'*Humicola sp*. recombinée selon l'une quelconque des revendications 18 à 31, qui est une lipase d'*Humicola lanuginosa*.
